# EUROPEAN PATENT APPLICATION

(11) **EP 4 218 997 A1**
(43) Date of publication of application: **02.08.2023**
(21) Application number: 22862401.1
(22) Date of filing: 05.07.2022
(51) Int. Cl.: B01J 8/00, B01J 8/06, B01D 5/00, C07C 2/08, C07C 11/04, C07C 11/107

(54) **BUBBLE COLUMN REACTOR**

(30) Priority: 10.12.2021 KR 20210177025
(71) Applicant: LG CHEM, LTD., Yeongdeungpo-gu, Seoul 07336 (KR)
(72) Inventor: HWANG, Moon Sub, Daejeon 34122 (KR); LEE, Hong Min, Daejeon 34122 (KR); LEE, Jeong Seok, Daejeon 34122 (KR); SONG, Jong Hun, Daejeon 34122 (KR); YOUK, Kyung Seog, Daejeon 34122 (KR)
(74) Representative: Hoffmann Eitle
(86) International application number: PCT/KR2022/009666
(87) International publication number: WO 2023/106533

(57) **Abstract**

Provided is a bubble column reactor including a reaction zone in which a reaction of a gaseous reactant is carried out in a liquid reaction medium; a disengaging section provided above the reaction zone and into which a first gas stream rising from the reaction zone is introduced; and a condensation zone provided above the disengaging section and into which a second gas stream rising from the disengaging section is introduced, wherein a diameter of the condensation zone is greater than a diameter of the disengaging section.

## Description

### [Technical Field]

### Cross-reference to related application

The present application claims priority to Korean Patent Application No. 10-2021-0177025, filed on December 10, 2021, the entire contents of which are incorporated herein for all purposes by this reference.

### Technical field

The present invention relates to a bubble column reactor, and more particularly, to an oligomer production apparatus for reducing the amount of entrained solids and liquids inside a reactor during oligomer production, thereby improving stabilization of an overall process.

### [Background Art]

Alpha-olefins are widely used commercially as important materials used in comonomers, detergents, lubricants, and plasticizers. In particular, 1-hexene and 1-octene have been widely used as comonomers for controlling the density of polyethylene in the production of linear low-density polyethylene (LLDPE).

Alpha olefins are typically prepared through oligomerization of ethylene. As a reactor type in which the ethylene oligomerization reaction is carried out, a bubble column reactor performing an oligomerization reaction of ethylene (trimerization reaction or tetramerization reaction) through contact with a reaction zone including a liquid reaction medium including a catalyst using gaseous ethylene as a reactant has been used.

In the case of a bubble column reactor, gas, which is a reactant, is mixed with a liquid reaction medium in the reaction zone to exist in two phases. As a result of a catalytic reaction, a small amount of polymer is produced as a by-product, which floats in the liquid reaction medium. At this time, entrainment of the solid polymer and the liquid reaction medium inevitably occurs due to a rate at which a large amount of gaseous reactant is introduced into the reaction zone in the form of a large amount of bubbles.

Due to such entrainment, a by-product polymer accumulates not only on an inner wall of the reactor but also on downstream process devices such as condensers, pipes, and valves, thereby causing fouling. As such, fouling occurring in the downstream process devices of the reactor causes deterioration of the devices and mechanical damage, and in the worst case scenario, it may be necessary to shut down the operation of the entire process, which resultantly increases costs incurring in a washing process, as well as reduces the productivity according to a reduction in the operating time.

Therefore, in order to solve the above problems, there is a need for research to reduce the amount of entrainment of solids and liquids containing polymers in the bubble column reactor.

### [Disclosure]

### [Technical Problem]

In order to solve the problems mentioned in the background of the present invention, an object of the present invention is to provide a bubble column reactor for preventing by-products including polymer materials, other than a desired product in the reactor, from being entrained, thereby improving stabilization of an overall process.

### [Technical Solution]

In one general aspect, a bubble column reactor includes: a reaction zone in which a reaction of a gaseous reactant is carried out in a liquid reaction medium; a disengaging section provided above the reaction zone and into which a first gas stream rising from the reaction zone is introduced; and a condensation zone provided above the disengaging section and into which a second gas stream rising from the disengaging section is introduced, wherein a diameter of the condensation zone is greater than a diameter of the disengaging section.

### [Advantageous Effects]

According to the bubble column reactor of the present invention, a condensation zone is provided above the reaction zone of the bubble column reactor, thereby preventing fouling for the condenser itself due to entrainment, compared to a case in which a separate condenser is provided outside the bubble column reactor of the related art.

Meanwhile, by forming a diameter of the condensation zone of the bubble column reactor to be larger than a diameter of the disengaging section provided between the reaction zone and the condensation zone, a gas rising rate in the condensation zone may be reduced to improve a precipitation effect of non-vapor. In addition, a path of a gas is changed by a vortex generated in the condensation zone having a relatively larger diameter than that of the disengaging section, so that uniform mixing in the condensation zone and resultantly uniform condensation may be obtained.

Meanwhile, a uniform temperature distribution in the condensation zone may be implemented through the arrangement of the cooling coil in the condensation zone, whereby a solvent and polymer in the gaseous stream may be effectively condensed and refluxed to the reaction zone.

Accordingly, the stability of the entire process may be improved by reducing the amount of entrained non-vapor of solids and liquids, an operation stop cycle of the reactor may be effectively increased by fundamentally preventing the occurrence of fouling in the downstream process devices of the reactor, and energy cost may be reduced by preventing an efficiency degradation due to fouling of the downstream process devices.

### [Description of Drawings]

FIGS. 1 and 2 are views illustrating a bubble column reactor and a related process flow according to an embodiment of the present invention.
FIGS. 3 and 4 are a view showing a bubble column reactor of the related art and a related process flow diagram according to the related art.

### [Best Mode]

The terms and words used in the description and claims of the present invention are not to be construed as general or dictionary meanings but are to be construed as meanings and concepts meeting the technical ideas of the present invention based on a principle that the inventors may appropriately define the concepts of terms in order to describe their own inventions in the best mode.

The term "stream" in the present invention may refer to a flow of a fluid in a process, and may also refer to a fluid flowing through a transfer line (pipe) itself. Specifically, the "stream" may refer to both the fluid flowing through the pipe connecting respective devices to each other itself and the flow of the fluid at the same time. In addition, the fluid may refer to one or more of gas, liquid and solid.

The term "C#" in which "#" is a positive integer in the present invention refers to all hydrocarbons having # carbon atoms. Accordingly, the term "C10" refers to a hydrocarbon compound having 10 carbon atoms. In addition, the term "C#+" refers to all hydrocarbon molecules having # or more carbon atoms. Accordingly, the term "C10+" refers to a mixture of hydrocarbons having 10 or more carbon atoms.

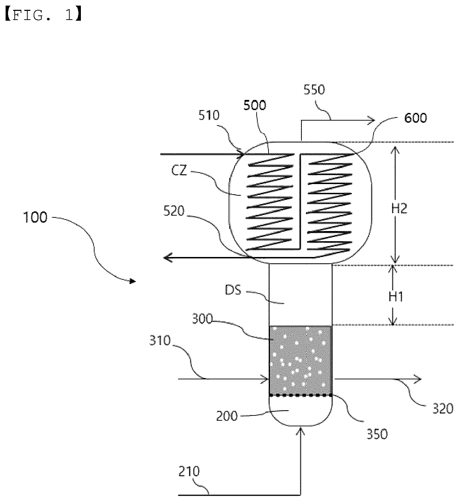

Hereinafter, the present invention will be described in more detail with reference to FIG. 1 in order to help the understanding of the present invention.

A bubble column reactor 100 according to an embodiment of the present invention may include a reaction zone in which a reaction of a gaseous reactant is carried out in a liquid reaction medium, a disengaging section provided above the reaction zone and into which a first gas stream rising from the reaction zone is introduced, and a condensation zone provided above the disengaging section and into which a second gas stream rising from the disengaging section is introduced, wherein a diameter of the condensation zone is greater than a diameter of the disengaging section.

According to an embodiment of the present invention, the bubble column reactor 100 may be for preparing an oligomer product by performing an oligomerization reaction of a gaseous reactant including a monomer in a liquid reaction medium of a solvent and a catalyst.

More specifically, the bubble column reactor 100 may include the reaction zone 300, and through one or more reaction medium supply lines 310 connected to one side of the reaction zone 300, the reaction medium may be supplied to the reaction zone 300. Here, the reaction medium may include a catalyst, a co-catalyst, and a solvent. The catalyst, the co-catalyst, and the solvent may be supplied through separate reaction medium supply lines 310, respectively, and two or more reaction medium components may be mixed and supplied to the reaction zone 300 through the reaction medium supply line 310.

According to an embodiment of the present invention, the monomer may include an ethylene monomer. As a specific example, the gaseous reactant including the ethylene monomer may be supplied into a down chamber 200 of the bubble column reactor 100 to be described later to produce a desired alpha olefin product through an oligomerization reaction.

The solvent may include one or more selected from the group consisting of n-pentane, n-hexane, n-heptane, cyclohexane, methylcyclohexane, octane, cyclooctane, decane, dodecane, benzene, xylene, 1,3,5-trimethylbenzene, toluene, ethylbenzene, chlorobenzene, dichlorobenzene, and trichlorobenzene.

The catalyst may include a transition metal source. The transition metal source may be a compound including one or more selected from the group consisting of, for example, chromium (III) acetylacetonate, chromium (III) chloride tetrahydrofuran, chromium (III) 2-ethylhexanoate, chromium (III) tris(2,2,6,6-tetramethyl-3,5-heptanedionate), chromium (III) benzoylacetonate, chromium (III) hexafluoro-2,4-pentaindionate, chromium (III) acetate hydroxide, chromium (III) acetate, chromium (III) butyrate, chromium (III) pentanoate, chromium (III) laurate, and chromium (III) stearate.

The co-catalyst may include one or more selected from the group consisting of, for example, trimethyl aluminum, triethyl aluminum, triisopropyl aluminum, triisobutyl aluminum, ethyl aluminum sesquichloride, diethylaluminum chloride, ethyl aluminum dichloride, methylaluminoxane, modified methylaluminoxane, and borate.

Meanwhile, in the reaction zone 300 of the bubble column reactor 100, the oligomerization reaction with the monomer may be performed in a liquid reaction medium including a catalyst, a co-catalyst, and a solvent. As described above, a zone composed of a reaction medium in which an oligomerization reaction of a monomer is performed may be defined as the reaction zone 300. The oligomerization reaction may refer to a reaction in which a monomer is oligomerized. Depending on the number of monomers to be polymerized, the oligomerization reaction may be called trimerization and tetramerization, which are collectively called multimerization.

The alpha olefin is widely used commercially as an important material used in a comonomer, detergent, lubricant, plasticizer, etc. In particular, 1-hexene and 1-octene are commonly used as a comonomer for controlling the density of polyethylene in the production of linear low-density polyethylene (LLDPE). The alpha olefins such as 1-hexene and 1-octene may be prepared through, for example, trimerization or tetramerization of ethylene.

The bubble column reactor 100 may include a product discharge line 320 connected to the reaction zone 300 and provided on the other side of the reaction medium supply line, and a product including alpha olefin, which is a product of the oligomerization reaction, may be discharged through the product discharge line. That is, the product discharge line 320 may include an oligomer product generated through an oligomerization reaction and a solvent, and the oligomer product and the solvent may be separated through an additional separation device. The separated solvent may be reused in the oligomer production process. Also, for example, when the oligomerization reaction is performed using an ethylene monomer as the monomer, the oligomer product may include 1-hexene and 1-octene. The supply of the reaction medium to the reaction zone 300 and the discharge of the product from the reaction zone 300 may be performed continuously.

Meanwhile, the gaseous reactant including the monomer for the oligomerization reaction may be supplied into the down chamber 200 located at the lower portion of the bubble column reactor 100 through a gaseous reactant supply pipe 210, and then pass through a dispersion plate 350 to be supplied to the reaction zone 300 including the liquid reaction medium.

That is, the dispersion plate 350 may be provided between the down chamber 200 and the reaction zone 300, and the gaseous reactant, e.g., the gaseous monomer, may be uniformly distributed and supplied to the reaction zone 300 including the reaction medium from the down chamber 200 through holes formed at equal intervals along the center and circumference of the dispersion plate 350.

The gaseous reactant flows into the reaction zone 300 containing the liquid reaction medium through the dispersion plate 350 and is dispersed at the same time, and turbulence is generated by a force of the dispersed gas, so that the liquid reaction medium and the gaseous reactant are naturally mixed. At this time, the dispersion force of the gaseous reactant flowing into the reaction zone 300 through the dispersion plate 350 may be maintained to be greater than a head pressure acting downwardly from the liquid reaction medium, so that the liquid reaction medium may remain in the reaction zone 300.

Meanwhile, as described above, the gaseous reactant supplied to the reaction zone 300 of the bubble column reactor 100 may be catalyzed in the liquid reaction medium in which a solvent and a catalyst are present, and specifically, the catalytic reaction may be an oligomerization reaction. In this case, the gaseous reactant and the reaction medium in the reaction zone 300 are mixed with each other to exist in two phases. Meanwhile, in the reaction zone 300, a small amount of polymer may be produced as a by-product as a result of the catalytic reaction of a gaseous reactant, which floats in the liquid reaction medium. Meanwhile, a large amount of gaseous reactant is introduced into the reaction zone 300 and unreacted gaseous reactant passes through a reaction medium in a gaseous state to rise. Entrainment of the solid polymer and the liquid reaction medium may take place due to a flow rate and velocity of the rising gaseous reactant. That is, when the unreacted gaseous reactant moves upwardly through the reaction zone 300, not only the unreacted gaseous reactant but also the polymer and partially vaporized solvent, which are by-products, move upwardly together. In this case, the entrained polymer is deposited in the downstream process device of the bubble column reactor 100 due to the adhesiveness of the polymer, thereby causing a fouling phenomenon that inhibits flowability of the fluid.

Accordingly, the bubble column reactor 100 according to an embodiment of the present invention may include a disengaging section DS and a condensation zone CZ provided above the reaction zone 300, so that by-products containing a polymer are prevented from being entrained, thereby preventing fouling in the downstream process device and improving the stability of the entire process.

More specifically, a first gaseous stream from the reaction zone 300 may be introduced into the disengaging section DS. The first gaseous stream may include a vaporized solvent and entrained polymer as a mixed gas in addition to the unreacted gaseous reactant. The first gas stream including the unreacted gaseous reactant and the mixed gas may be reduced in an upward movement rate by passing through the disengaging section DS and a portion of the entrained polymer and the solvent, in particular, a portion of the polymer non-vapor that is a solid material having a relatively heavy specific gravity, may be preferentially precipitated into the reaction zone 300.

Meanwhile, a height H1 of the disengaging section DS, that is, a length from an upper surface of the reaction zone 300 to an upper surface of the disengaging section DS, may be 10% to 40% of a total length of the bubble column reactor 100. When the height H1 of the disengaging section DS is 10% or more of the total length of the bubble column reactor 100, sufficient time and space may be provided for precipitation of the entrained polymer and solvent in the first gas stream, and when the height H1 of the disengaging section DS is 40% or less, a space occupied by the bubble column reactor 100 may be minimized in addition to the efficient precipitation effect of the disengaging section DS.

Subsequently, the gas stream rising from the disengaging section DS may then be introduced as a second gas stream into the condensation zone CZ provided above the disengaging section DS.

The condensation zone CZ is a space in which an internal temperature is maintained to be lower than that of the disengaging section DS, and, while the second gas stream passes through the condensation zone CZ, the vaporized solvent and the entrained polymer in the second gas stream may be condensed and precipitated. In particular, the vaporized solvent may be liquefied by condensation, and the liquefied solvent may be precipitated and refluxed to the reaction zone 300.

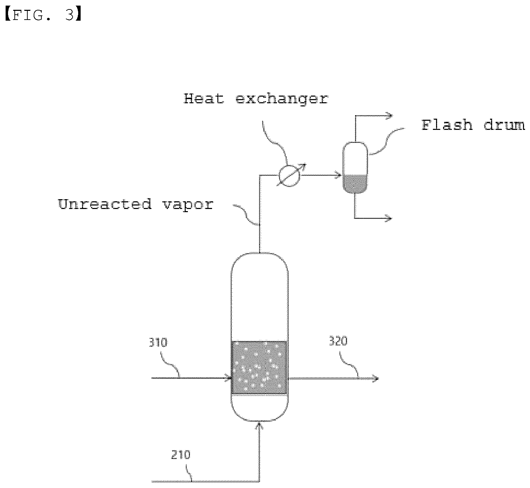

Meanwhile, referring to FIG. 3 showing a bubble column reactor of the related art, the bubble column reactor sequentially includes the down chamber, the reaction zone, and the disengaging section therein, but does not include a condensation zone as in the present invention inside the bubble column reactor. That is, the unreacted vapor discharged to the upper portion of the bubble column reactor was condensed through an external heat exchanger, and then the condensed components such as solvents and the unreacted gas reactants were separated through a separation device, for example, a flash drum. In this case, the entrained polymer component may be discharged as it is to the upper portion of the bubble column reactor and the polymer component may be deposited in the heat exchanger, the flash drum, and the pipe connecting them to cause frequent fouling.

That is, according to an embodiment of the present invention, since the condensation zone CZ is provided inside the bubble column reactor 100, the effect of quickly condensing and precipitating the mixed gas may be achieved, even without a separate facility such as a pipe, compared to the case in which a separate condenser is provided outside the bubble column reactor. Furthermore, the fouling problem in the pipe between the bubble column reactor and the external condenser due to the entrained polymer or the fouling problem in the condenser itself may be fundamentally solved.

Meanwhile, the bubble column reactor 100 may have a cylindrical shape having a circular cross-section, and cross-sections of the reaction zone 300, the condensation zone CZ, and the disengaging section DS may also have a circular shape. A diameter of the disengaging section DS may be the same as a diameter of the reaction zone 300, and a diameter of the condensation zone CZ may be larger than a diameter of the disengaging section DS. Here, the diameter of the condensation zone CZ specifically refers to a diameter of the cross-section of the condensation zone CZ, and the diameter of the disengaging section DS specifically refers to a diameter of the cross-section of the disengaging section DS.

When the diameter of the condensation zone CZ is larger than the diameter of the disengaging section DS, a path of the second gas stream passing through the disengaging section DS having a relatively narrow crosssectional area is changed in the inlet of the condensation zone CZ, thereby generating a vortex. Due to this vortex, the second gas stream may be mixed at the inlet of the condensation zone CZ and may be uniformly distributed and raised. Furthermore, since the diameter of the condensation zone CZ is increased, the rising speed of the rising second gas stream may be reduced, so that the condensation of the vaporized solvent and the precipitation effect of the entrained polymer are excellent. As the uniformly mixed second gas stream passes through the condensation zone CZ at a reduced rate, the condensation efficiency of the condensation zone CZ may be further increased.

Specifically, the diameter of the condensation zone CZ may be 1.3 times to 3 times the diameter of the disengaging section DS, and more specifically, 1.5 times to 2 times the diameter of the disengaging section DS. When the ratio of the diameters is 1.3 times or more, an excellent non-vapor precipitation effect may be achieved by uniform mixing of the aforementioned second gas stream and decreasing the rising rate. Meanwhile, when the ratio is 3 times or less, a minimum rising pressure for the second gas stream to pass through the condensation zone CZ may be maintained.

Meanwhile, the condensation zone CZ may include a cooling coil, and the cooling coil may be provided from an upper portion to a lower portion inside the condensation zone CZ in a wound state. The cooling coil is in the form of a pipe through which a refrigerant may flow, and the cooling coil may include a cooling coil inlet through which a refrigerant may be introduced into the cooling coil at an upper portion of the condensation zone CZ and a cooling coil outlet through which the refrigerant may be discharged to the cooling coil at a lower portion of the condensation zone CZ.

Meanwhile, a temperature of the refrigerant supplied from the upper portion of the condensation zone CZ of the present invention may be in the range of -10 °C to -5 °C and a temperature of the refrigerant discharged from the lower portion of the condensation zone CZ may be in the range of 0 °C to 5 °C.

Considering an oligomerization reaction temperature in the reaction zone 300, the temperature of the refrigerant supplied to the condensation zone CZ needs to be at least lower than -5 °C for condensation of the gaseous stream. In addition, the temperature of the supplied refrigerant needs to be -10 °C or higher according to an available reaction temperature range and the need for energy saving.

Meanwhile, in order to lower the temperature of a gaseous effluent stream 550 from the predetermined oligomerization reaction temperature, a difference between the supply temperature of the refrigerant and the temperature of the discharged refrigerant is preferably about 10 °C, and in this respect, the temperature of the discharged refrigerant may be in the range of 0 °C to 5 °C.

As the diameter of the condensation zone CZ is relatively increased, it is important to maintain an appropriate cooling temperature inside the condensation zone CZ. Accordingly, the cooling coil may include a first cooling coil 500 and a second cooling coil 600, wherein the first and second cooling coils may be wound and provided from the upper portion to the lower portion inside the condensation zone CZ.

Referring to FIG. 1, the first cooling coil 500 and the second cooling coil 600 according to an embodiment of the present invention may be connected in parallel in the condensation zone CZ, and a lower end of the first cooling coil 500 may extend upwardly to be connected to an upper end of the second cooling coil.

Specifically, an inlet 510 of the first cooling coil 500 for introducing a refrigerant may be provided at an upper portion of the first cooling coil 500, and the refrigerant introduced into the inlet 510 of the first cooling coil 500 may exchange heat with the second gas stream, while moving downwardly along the inside of the first cooling coil 500 wound in the form of a coil. The refrigerant that has moved to the lower portion of the first cooling coil 500 may be reintroduced to the upper portion of the second cooling coil 600. Then, the refrigerant may exchange heat with the second gas stream, while moving along the inside of the second cooling coil 600 wound in the form of a coil. The refrigerant may be discharged to the outside of the reactor through the outlet 520 of the second cooling coil 600 provided at a lower portion of the second cooling coil 600.

As such, the bubble column reactor 100 of the present invention includes the first and second cooling coils in the condensation zone CZ, so that, even if the diameter of the condensation zone CZ is larger than the diameter of the disengaging section DS, a sufficient heat exchange area between the cooling coil and the second gas stream may be secured in the condensation zone CZ, thereby achieving a uniform cooling temperature distribution in the condensation zone CZ. In addition, since the refrigerant supplied to the first cooling coil 500 may be used as the refrigerant of the second cooling coil 600, an effect of saving energy may be achieved.

Also, when natural precipitation of a portion of the entrained polymer having a relatively high specific gravity and vaporized solvent in the first gas stream takes place and the rest of the polymer and vaporized solvent rises in the disengaging section DS, a rising speed is reduced due to the increase in the diameter of the condensation zone CZ and the direction of movement is changed by the vortex. Therefore, uniform mixing and thus uniform condensation are made in the condensation zone CZ, and, meanwhile, a non-contact area (dead zone) is reduced, so that a phenomenon in which the polymer and the vaporized solvent are not precipitated or condensed but pass through the condensation zone CZ may be prevented.

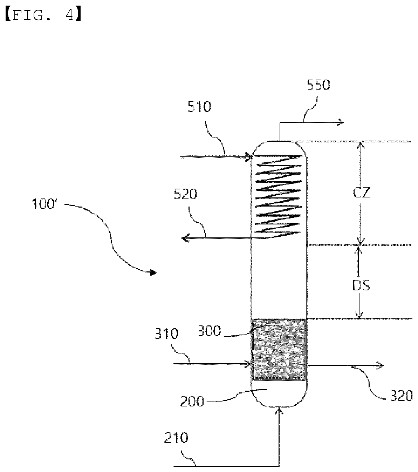

Meanwhile, referring to FIG. 4, when the condensation zone CZ of the bubble column reactor includes one row of cooling coils, condensation may take place around the cooling coil, but relatively less condensation takes place at a point far from the cooling coil, resulting in an increase in the non-contact area (dead zone). With such a one-row cooling coil of the related art, efficient condensation of the vaporized solvent contained in the second gas stream in the condensation zone CZ with an increased diameter and the precipitation efficiency of non-vapor are degraded, and thus, when the diameter of the condensation zone CZ is relatively increased, there is a problem in that it is difficult to maintain an appropriate cooling temperature inside the condensation zone CZ.

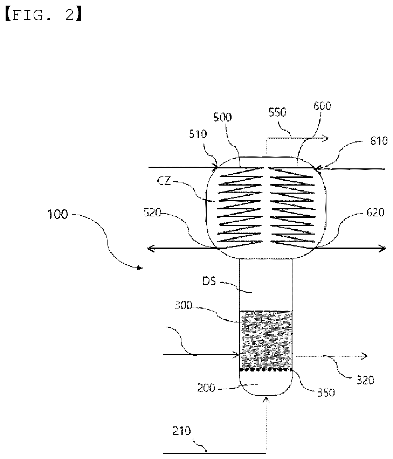

According to another embodiment of the present invention, the first cooling coil 500 and the second cooling coil 600 may be separated and spaced apart from each other. Specifically, referring to FIG. 2, the refrigerant may be introduced into the respective inlets 510 and 610 provided at the upper ends of the first cooling coil 500 and the second cooling coil 600, and the refrigerant may exchange heat with the second gas stream, while moving downwardly along the inside of the first and second cooling coils 500 and 600 wound in a coil form. Subsequently, the refrigerant may be discharged to the respective outlets 520 and 620 provided at the lower ends of the first and second cooling coils 500 and 600. In this case, a temperature deviation of the condensation zone CZ in the radial direction may be minimized, and the internal temperature of the condensation zone CZ may be more uniformly controlled.

Meanwhile, the ratio (H2/H1) of the height H2 of the condensation zone to the height H1 of the disengaging section may be 1.2 to 2.0. When the ratio of the height is within the range, an excellent non-vapor precipitation effect may be obtained because sufficient time and space necessary for precipitation of the entrained polymer and solvent in the first gas stream in the disengaging section DS are provided. As such, due to the excellent non-vapor precipitation effect in the condensation zone CZ, the height of the disengaging section DS may be reduced, thereby minimizing the space occupied by the bubble column reactor 100.

As such, in the case of an embodiment of the present invention, non-vapor such as the vaporized solvent and polymer in the second gas stream in the condensation zone CZ may be condensed and precipitated, and the gaseous effluent stream 550 including unreacted monomer, for example, gaseous ethylene, may be discharged to the top of the bubble column reactor 100. In this case, the gaseous effluent stream 550 may be discharged in a state in which most of the mixed gas (vaporized solvent and entrained polymer) included in the first effluent stream is removed.

Hereinabove, the bubble column reactor according to the present invention has been described and illustrated in the drawing. However, the description and the illustration of the drawing are for only essential components for understating the present invention, and processes and apparatuses not separately described and illustrated may be properly applicable and used for implementing the bubble column reactor according to the present invention, in addition to the processes and apparatuses described and illustrated in the drawing.

## Claims

1. A bubble column reactor comprising:
a reaction zone in which a reaction of a gaseous reactant is carried out in a liquid reaction medium;
a disengaging section provided above the reaction zone and into which a first gas stream rising from the reaction zone is introduced; and
a condensation zone provided above the disengaging section and into which a second gas stream rising from the disengaging section is introduced,
wherein a diameter of the condensation zone is greater than a diameter of the disengaging section.

2. The bubble column reactor of claim 1,
wherein the diameter of the condensation zone is 1.3 times to 3 times the diameter of the disengaging section.

3. The bubble column reactor of claim 1,
wherein the diameter of the condensation zone is 1.5 times to 2 times the diameter of the disengaging section.

4. The bubble column reactor of claim 1,
wherein the condensation zone includes a cooling coil,
wherein the cooling coil is provided from an upper portion to a lower portion inside the condensation zone in a wound state.

5. The bubble column reactor of claim 4,
wherein the cooling coil includes a first cooling coil and a second cooling coil,
wherein the first cooling coil and the second cooling coil are each provided from the upper portion to the lower portion inside the condensation zone in a wound state.

6. The bubble column reactor of claim 5,
wherein the first cooling coil and the second cooling coil are connected to each other in parallel, and
a lower end of the first cooling coil extends upwardly and is connected to an upper end of the second cooling coil.

7. The bubble column reactor of claim 6,
wherein the first cooling coil includes an inlet of the first cooling coil provided at an upper end, and
a temperature of a refrigerant introduced into the inlet of the first cooling coil is -10 °C to -5 °C.

8. The bubble column reactor of claim 5,
wherein the first cooling coil and the second cooling coil are spaced apart from each other, and
a refrigerant is introduced into respective inlet provided at upper ends of the first and second cooling coils, and the refrigerant is discharged through respective outlet provided at lower ends of the first and second cooling coils.

9. The bubble column reactor of claim 1,
wherein a ratio(H2/H1) of a height of the condensation zone(H2) to a height of the disengaging section(H1) is 1.2 to 2.0.

10. The bubble column reactor of claim 1,
wherein the gaseous reactant includes an ethylene monomer.
